# EUROPÄISCHE PATENTSCHRIFT

(11) **EP 1 299 707 B1**
(45) Veröffentlichungstag und Bekanntmachung des Hinweises auf die Patenterteilung: **29.12.2004**
(21) Anmeldenummer: 01962843.7
(22) Anmeldetag: 07.07.2001
(51) Int. Cl.: G01N 15/14, G06K 9/00

(54) **VERFAHREN ZUR UNTERSUCHUNG VON ZELLEN IN EINER KULTURFLÜSSIGKEIT**
METHOD FOR THE EXAMINATION OF CELLS IN A CULTURE MEDIUM
PROCEDE POUR L'EXAMEN DE CELLULES DANS UN LIQUIDE DE CULTURE

(30) Priorität: 10.07.2000 DE 10033268
(43) Veröffentlichungstag der Anmeldung: 09.04.2003
(73) Patentinhaber: Innovatis AG, 33607 Bielefeld (DE)
(72) Erfinder: BITTNER, Christoph, Herts, HP3 9PT (GB)
(74) Vertreter: Heiland, Karsten, Dipl.-Ing.
(86) Internationale Anmeldenummer: PCT/EP2001/007814
(87) Internationale Veröffentlichungsnummer: WO 2002/004924

(56) Entgegenhaltungen:
- EP-A- 0 391 674
- WO-A-99/35496
- WO-A-99/44593
- DE-A- 4 032 002
- US-A- 2 850 239
- US-A- 4 776 697
- US-A- 5 188 968
- RAMIC A J ET AL: "Temporary droplet-size hysteresis in immiscible polymer blends" POLYMER, ELSEVIER SCIENCE PUBLISHERS B.V, GB, Bd. 41, Nr. 16, Juli 2000 (2000-07), Seiten 6263-6270, XP004195895 ISSN: 0032-3861
- BURMEISTER J S ET AL: "Application of total internal reflection fluorescence microscopy to study cell adhesion to biomaterials" BIOMATERIALS, ELSEVIER SCIENCE PUBLISHERS BV., BARKING, GB, Bd. 19, Nr. 4-5, 1. März 1998 (1998-03-01), Seiten 307-325, XP004122210 ISSN: 0142-9612

## Beschreibung

Die vorliegende Erfindung betrifft ein Verfahren zur Untersuchung von Zellen in einer Kulturflüssigkeit, insbesondere zur In-situ-Mikroskopie in einem Bioreaktor, gemäß dem Oberbegriff des Anspruchs 1, sowie eine Vorrichtung zur Durchführung dieses Verfahrens.

Ein solches Verfahren wird beispielsweise durch die DE 40 32 002 C2 offenbart und ist insbesondere zum Einsatz in Bioreaktoren vorteilhaft, die auch bei der Kultivierung von Zellen im industriellen Maßstab verwendet werden. Zur automatischen Regelung des Kultivierungsprozesses der Zellen im Reaktor werden Meßwerte benötigt, die Aufschluß über den Zustand der Kulturflüssigkeit geben. Grundsätzlich ist dieses Verfahren nicht auf organische Zellen beschränkt, sondern kann auch zur Untersuchung anderer, nicht-organischer Partikel in einer Flüssigkeit, wie etwa einer Ölsuspension oder dergleichen, verwendet werden. Daher ist der Begriff der Zelle hier im weitesten Sinne zu verstehen, obwohl im folgenden auf organische Zellen Bezug genommen wird.

Bei dem bekannten Verfahren werden die Zellen mikroskopisch abgebildet, und die Abbildung wird durch eine automatische Bildverarbeitung erfaßt. Das untersuchte Probenvolumen ist in Richtung der optischen Achse des Mikroskops durch Fenster definiert, die einen problemlosen Einblick in das Probenvolumen gewähren.

Von Interesse sind insbesondere die Zellkonzentration, die Größe und die Morphologie der Zellen. Durch die In-situ-Mikroskopie läßt sich ferner die Zellgrößenverteilung bestimmen, so daß auf die Biotrockenmasse geschlossen werden kann. Diese Parameter werden durch das beschriebene Verfahren unmittelbar geliefert, während Off-line-Methoden, die eine manuelle Probenentnahme und Analyse erfordern, für eine zufriedenstellende Regelung des Kultivierungsprozesses unbrauchbar sind.

Es erweist sich jedoch als schwierig, detaillierte Aussagen über die Zellpopulation in dem Bioreaktor zu erhalten, da die Bildverarbeitung es nur in eingeschränktem Maße erlaubt, alle Zellen im Probenvolumen zuverlässig zu erfassen. Damit möglichst viele Zellen untersucht werden können, muß der Abstand der Fenster und somit die Dicke des Probenvolumens groß gewählt werden. Dies ist wiederum problematisch, da aufgrund der begrenzten Schärfentiefe des Objektivs der scharf abgebildete Bereich sehr schmal ist, so daß nur wenige Zellen scharf abgebildet werden und daher das Bildverarbeitungssystem bei jedem Meßzyklus nur wenige Zellen erfaßt. Die Unterscheidung einzelner Zellen ist auch dann schwierig, wenn sich hintereinander schwimmende Zellen gegenseitig verdecken. Damit die Abbildung noch sinnvoll ausgewertet werden kann, ist eine aufwendige Bildverarbeitungssoftware notwendig, die jedoch nicht immer zuverlässige Ergebnisse liefert. Zudem kann es bei manchen Untersuchungsmethoden erforderlich sein, die Zellen zur Abbildung länger zu belichten. Dies ist jedoch nicht möglich, wenn die Zellen sich frei im Probenvolumen bewegen können.

Falls andererseits das Probenvolumen in Richtung der optischen Achse so schmal gewählt wird, daß seine Dicke annähernd der Schärfentiefe des Mikroskopobjektivs entspricht, führt dies dazu, daß sich nur sehr wenige Zellen innerhalb des Probenvolumens aufhalten und untersucht werden können. Ist das Probenvolumen sehr schmal, wird zudem ein einwandfreies Durchströmen der Kulturflüssigkeit zwischen den Fenstern behindert.

RAMIC A J ET AL; "Temporary droplet-size hysteresis in immiscible polymer blends"; POLYMER, ELSEVIER SCIENCE PUBLISHERS B.V, GB, Vol.41, No. 16, pages 6263 - 6270;July 2000 offenbart eine Vorrichtung zur Untersuchung von Tröpfchenbildung, mit einem X-60 optischen Mikroskop, einer CCD-Videokamera, einem Beobachtungsfenster und einer Scherkraßzelle, die aus zwei parallelen Platten mit einem justierbaren, dazwischenliegenden, planaren Hohlraum besteht. Mittels einer Steuereinheit wird der Plattenabstand über einen Computer geregelt. Eine Bilderfassung erfolgt durch "Vision Explorer Software" und Tröpfchendurchmesser werden mit "Labview Software - IMAQ" berechnet.

Aufgabe der Erfindung ist es daher, ein Verfahren der eingangs genannten Art aufzuzeigen, das es ermöglicht, während eines Meßzyklus eine relativ große Anzahl von Zellen zuverlässig zu erfassen, ohne daß die oben beschriebenen Probleme auftreten, sowie eine Vorrichtung zu dessen Durchführung zu schaffen.

Diese Aufgabe wird erfindungsgemäß durch ein Verfahren gemäß Anspruch 1 sowie eine Vorrichtung gemäß Anspruch 9 gelöst.

Bei dem erfindungsgemäßen Verfahren zur In-situ-Mikroskopie wird die Dicke des Probenvolumens der Größe der zu untersuchenden Zellen in der Weise angepaßt, daß der Abstand der Fenster allmählich verringert wird, während die Abbildungsgröße der Zellen durch das Bildverarbeitungssystem kontrolliert wird. Wird der Abstand der Fenster so schmal, daß sie die Zellen von beiden Seiten berühren. werden die Zellen zusammengedrückt und abgeplattet, so daß die Abbildungsgröße der Zellen an diesem Punkt anzuwachsen beginnt. Dieses Anwachsen der Abbildungsgröße wird durch die Bildverarbeitung registriert, und der Abstand der Fenster wird auf einen konstanten Abstandswert eingestellt, an dem die Zunahme der Abbildungsgröße durch die Abplattung gerade einzusetzen beginnt.

Bei diesem Abstandswert berühren die Fenster die Zellen im Probenvolumen von beiden Seiten, so daß die Zellen geringfügig eingeklemmt sind und die Dicke des Probenvolumens etwa der Zelldicke entspricht. Auf diese Weise kann das Probenvolumen sehr klein gemacht werden, während sich eine relativ große Anzahl von Zellen darin befindet und untersucht werden kann. Diese Zellen befinden sich alle in einer Objektebene senkrecht zur optischen Achse des Mikroskops und innerhalb dessen Schärfentiefe, so daß alle Zellen gut abgebildet werden. Ferner wird vermieden, daß die Abbildungen der Zellen einander überlappen, so daß alle Zellen einzeln identifizierbar sind. Da die Zellen in dieser Lage zwischen den Fenstern gehalten werden, lassen sich auch Abbildungen mit längeren Belichtungszeiten durchführen, falls dies erforderlich sein sollte.

Da beim Zusammenfahren der Fenster die Gefahr besteht, daß die Zellen zerdrückt werden, muß dieser Vorgang mit großer Präzision durchgeführt werden. Wird eine Abplattung der Zellen registriert, so wird der Abstand auf einen Wert zurückgefahren, an dem gerade noch keine Abplattung der Zellen aufgetreten ist. An diesem Punkt wird die Untersuchung der Zellen vorgenommen.

Falls sich Zellen unterschiedlicher Größen in der Kulturflüssigkeit befinden, lassen sich vorzugsweise durch die Bildverarbeitung Zellen verschiedener Größen klassifizieren, so daß sich die Zellen einer ausgewählten Größenklasse zur Bestimmung der Dicke des Probenvolumens heranziehen lassen. Dies bedeutet, daß der Abstand der Fenster an dem Punkt, an dem die Abbildungsgröße der Zellen dieser Größenklasse anzuwachsen beginnt, in der oben beschriebenen Weise auf einen konstanten Wert eingestellt wird. Die Abbildungsgröße bzw. die Abplattung der übrigen Zellen wird hierbei außer acht gelassen. Wird die Dicke des Probenvolumens beispielsweise entsprechend den größten Zellen eingestellt. so tritt während der Verringerung des Fensterabstandes keine Abplattung der übrigen Zellen auf, so daß diese sich relativ frei im Probenvolumen befinden. Stellt man den Wandabstand entsprechend kleinerer Zellen ein, so werden die größeren Zellen während des Zusammenfahrens zerstört. Die Abbildungen dieser zerstörten Zellen können durch die Bildverarbeitung identifiziert und bei der Untersuchung außer acht gelassen werden, so daß nur die kleineren Zellen analysiert werden.

Darüber hinaus ist es vorzugsweise möglich; Zellen zu untersuchen, die an plättchenförmigen Carriern anhaften. Bei diesen frei in der Kulturflüssigkeit schwimmenden Carriern handelt es sich um Polystyrolplättchen mit einem Durchmesser von ca. 0,1 bis 0,2 mm und einer Dicke von 20 µm. Diese Carrier tragen beispielsweise Säugerzellen, die sich bevorzugt auf Oberflächen ansiedeln. Die auf den Carriern haftenden Zellen lassen sich leicht beobachten, indem die Fenster der Probenkammer in der erfindungsgemäßen Weise zusammengefahren werden. Während dieses Vorgangs orientieren sich die Carrier so, daß sie flach zwischen den Fenstern liegen. Beobachtet man die Abbildungsgröße der Zellen, die sich auf einer Carrierseite befinden, also beispielsweise dem Mikroskopobjektiv zugewandt sind. so läßt sich der Abstand der Fenster in der beschriebenen Weise einstellen, indem dies so weit zusammengefahren werden, bis eine Abplattung der abgebildeten Zellen einsetzt. Der eingestellte Meßabstand entspricht also etwa der Dicke des Carriers zuzüglich des zweifachen Zelldurchmessers. Vorzugsweise wird während dieses Vorgangs die Abbildungsebene des Mikroskopobjektivs in die Zellschicht verlagert, die untersucht werden soll, und die Schärfentiefe wird so gering gewählt, daß ausschließlich Zellen in dieser Lage erfaßt werden. Die Zellen auf der gegenüberliegenden Oberflächenseite des Carriers werden somit nicht abgebildet, so daß bei der Bildverarbeitung keine Probleme durch überlappende Abbildungen hintereinander angeordneter Zellen auftreten. Die Schärfenebene kann zwischen den beiden Zellschichten auf den gegenüberliegenden Seiten der Carrier verlagert werden, so daß insgesamt alle Zellen im Probenvolumen getrennt von einander beobachtet werden können.

Die Schärfentiefe des Mikroskopobjektivs läßt sich vorzugsweise dadurch verändern. daß die numerische Apertur des Mikroskopobjektivs verändert wird. beispielsweise durch eine Blende.

Aufgrund der hohen erforderlichen Präzision der Abstandsbestimmung ist es relativ zeitaufwendig, den Abstand bei jedem Meßzyklus aufs Neue zu bestimmen. Bevorzugt wird daher in einem Meßzyklus der Abstandswert für eine bestimmte Zellart gespeichert und kann bei nachfolgenden Meßzyklen abgerufen werden, so daß der Abstand der Fenster unmittelbar auf den gespeicherten Wert eingestellt werden kann.

Befinden sich einerseits Carrier mit anhaftenden Zellen und andererseits freischwimmende Zellen in der Kulturflüssigkeit, so trifft das Bildverarbeitungssystem vorzugsweise eine Entscheidung darüber, ob sich ein Carrier oder Zellen im Probenvolumen befinden, damit der Fensterabstand entsprechend gewählt werden kann. Befindet sich beispielsweise ein Carrier im Probenraum, so wird ein vorbestimmter Abstandswert für den Carrier abgerufen und die Dicke in entsprechender Weise eingestellt. In der gleichen Weise wird die Dicke an eine Zelle angepaßt, sofern sich ausschließlich Zellen im Probenraum befinden. Vorzugsweise wird zumindest eines der Fenster vor oder nach dem Meßvorgang durch einen Wischer gereinigt, die an den transparenten Flächen anhaftende Zellen beseitigt.

Eine Vorrichtung zur Durchführung des erfindungsgemäßen Verfahrens wird durch Anspruch 9 offenbart. Die Unteransprüche 10 bis 12 offenbaren vorteilhafte Ausgestaltungen dieser Vorrichtung.

Im folgenden wird ein bevorzugtes Ausführungsbeispiel der Erfindung anhand der Zeichnungen näher erläutert.
Fig. 1 zeigt schematisch eine Ausführungsform einer Vorrichtung zur Durchführung des erfindungsgemäßen Verfahrens;
Fig. 2 bis 4 zeigen Detailansichten des Probenvolumens der Vorrichtung aus Fig. 1;
Fig. 5 zeigt ein Diagramm zur Erläuterung der Abhängigkeit der Abbildungsgröße von der Dicke des Probenvolumens;
Fig. 6 bis 8 zeigen Abbildungen des Probenvolumens entsprechend den Figuren 2 bis 4;
Fig. 9 bis 11 zeigen das Probenvolumen entsprechend den Figuren 2 bis 4 mit Zellen unterschiedlicher Großen;
Fig. 12 und 13 zeigen das Probenvolumen mit einem Carrier; und
Fig. 14 und 15 zeigen das Probenvolumen mit einem Wischer.

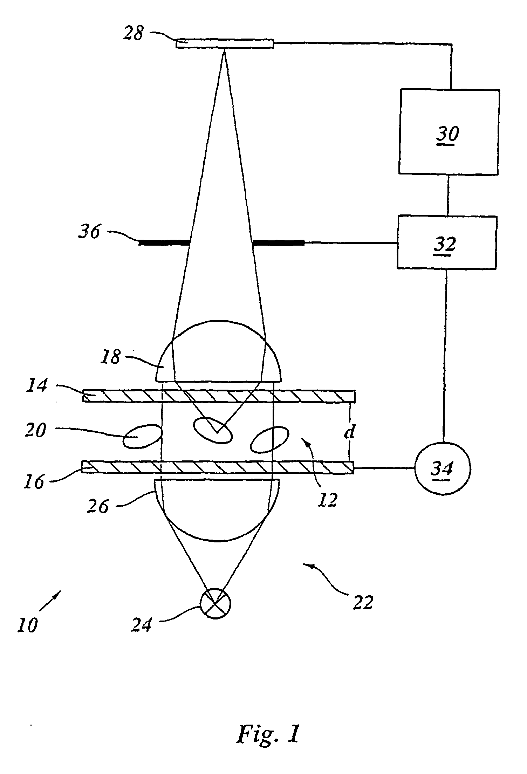

Die in Fig. 1 gezeigte Vorrichtung 10 zur In-situ-Mikroskopie von Zellen in einer Kulturflüssigkeit umfaßt ein Probenvolumen 12 zwischen zwei Fenstern 14,16, die senkrecht zur optischen Achse eines Mikroskopobjektivs 18 ausgerichtet sind, das zur Abbildung von Zellen 20 innerhalb des Probenvolumens 12 dient. Bei den Fenstern 14,16 handelt es sich im einfachsten Fall um Glasplatten. Die hier dargestellte Ausführungsform dient vorwiegend zur Untersuchung organischer Zellen 20; grundsätzlich ist die vorliegende Erfindung jedoch nicht auf diese beschränkt, sondern eignet sich auch zur Untersuchung nicht-organischer Partikel, die frei in der Flüssigkeit schwimmen.

Das Probenvolumen 12 wird durch eine Beleuchtungsanordnung 22 mit einer Lichtquelle 24 und einem Kondensator 26 im sogenannten Durchlichtverfahren durchleuchtet. Im hier gezeigten Fall handelt es sich um eine Hellfeld-Anordnung, es sind jedoch auch beliebige andere Beleuchtungsanordnungen verwendbar. Falls die Beleuchtung von der gleichen Seite erfolgt, auf der sich das Objektiv 18 befindet, wie es bei einer Auflicht-Anordnung der Fall ist, so muß das gegenüberliegende Fenster nicht zwangsläufig transparent sein, sondern es kann sich im Prinzip auch um eine lichtundurchlässige Rückwand der Probenkammer handeln.

Das Mikroskopobjektiv 18 bildet die Zellen 20 auf einem elektronischen Bildaufnehmer 28 ab, der an ein Bildverarbeitungssystem 30 angeschlossen ist, das zur elektronischen Erfassung und Verarbeitung der Abbildung dient. Ferner ist an das Bildverarbeitungssystem 30 eine Steuereinheit 32 zur Ansteuerung eines Stellmotors 34 angeschlossen, der zum linearen Verstellen des Fensters 16 dient, die das Probenvolumen 12 auf der dem Mikroskopobjektiv 18 abgewandten Seite dient. Die Dicke d des Probenvolumens 12 in Richtung der optischen Achse kann somit durch den Stellmotor 34 verändert werden. Darüber hinaus ist zwischen dem Mikroskopobjektiv 18 und dem Bildaufnehmer 28 eine verstellbare Blende 36 angeordnet, die ebenfalls durch die Steuereinheit 32 angesteuert wird.

Die Schärfentiefe des Objektivs 18 ist durch dessen numerische Apertur bestimmt. Ist die numerische Apertur hoch, so ist die Schärfentiefe gering, d. h, es wird nur ein sehr schmaler Bereich innerhalb des Probenvolumens 12 scharf abgebildet. Die numerische Apertur läßt sich verändern, in dem die Blende 36 geöffnet oder geschlossen wird. Damit eine große Anzahl von Zellen 20 innerhalb des Probenvolumens 12 scharf abgebildet werden kann, wird die Dicke d vor der Analyse so weit verringert, bis sich alle Zellen 20 innerhalb einer einzigen Lage zwischen den Fenstern 14.16 befinden.

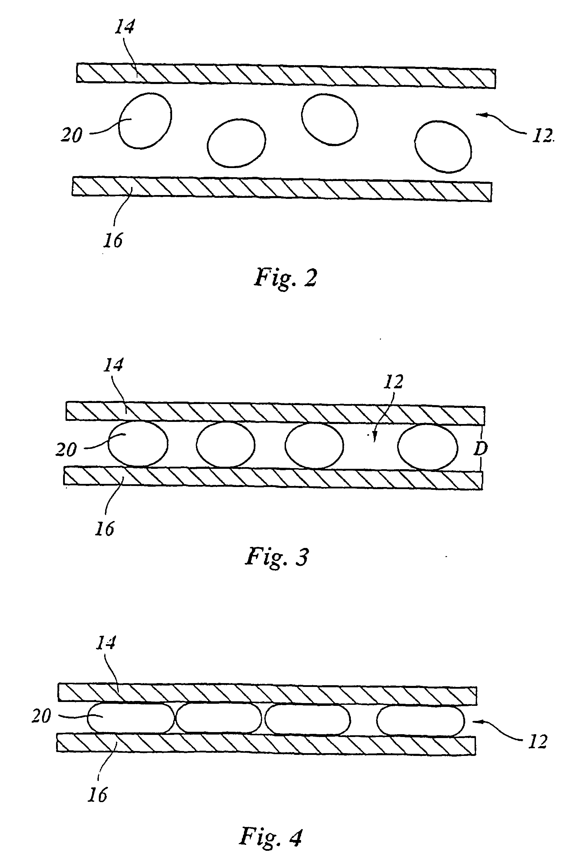

Dieser Vorgang ist in den Figuren 2 bis 4 dargestellt. Fig. 2 entspricht etwa der Situation aus Fig. 1, in der die Kulturflüssigkeit das Probenvolumen 12 frei durchströmen kann und die Zellen 20 sich frei bewegen können. Durch den Stellmotor 34 wird die Dicke d entsprechend Fig. 3 verringert, bis die Situation aus Fig. 4 erreicht ist, in der die Zellen durch den Anpreßdruck der Fenster 14.16 deutlich abgeplattet werden. Da die Abbildung der Zellen 20 durch das Bildverarbeitungssystem 30 ständig kontrolliert wird, gibt diese in dem Moment, in dem eine Abplattung registriert wird, ein Signal an die Steuereinheit 32, die den Stellmotor 34 in solcher Weise in der entgegengesetzten Richtung ansteuert, so daß wieder der Abstandswert D aus Fig. 3 erreicht wird, in der die Fenster 14.16 die Zellen 20 gerade berühren und noch keine Abplattung eingetreten ist. Dieser Abstand D ist zur Analyse der Zellen 20 optimal, so daß das in Fig. 3 gehaltene Bild zur Analyse der verschiedenen Zellparameter wie beispielsweise Zellkonzentration, Größe, Morphologie und Vitalität herangezogen wird.

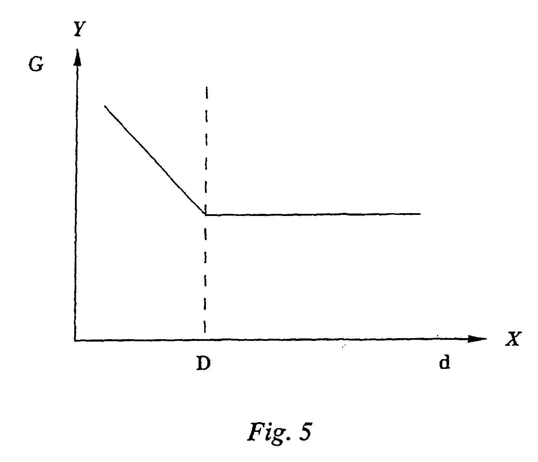

Fig. 5 illustriert den Vorgang der sukzessiven Verringerung der Dicke d des Probenvolumens 12 anhand eines Diagramms. Der Durchmesser G der Abbildung einer einzelnen Zelle 20 ist hier gegen den Abstand d der Fenster 14,16 aufgetragen. Wird der Abstand d mit einem großen Wert beginnend allmählich verringert, so bleibt der scheinbare Zelldurchmesser G zunächst konstant, bis die Zellen 20 schließlich entsprechend Fig. 3 zwischen den Fenstern 14,16 eingeklemmt werden. An diesem Punkt, der dem Abstandswert D entspricht, beginnt der Durchmesser G mit abnehmendem d stark anzuwachsen. Da während der Bewegung des Fensters 16 der Parameter G durch das Bildverarbeitungssystem 30 permanent überwacht wird, läßt sich dieser Punkt genau bestimmen, so daß die Dicke d genau auf den Abstandswert D eingestellt werden kann, an welchem die Abplattung gerade einzusetzen beginnt. Wird also dieser Punkt überfahren, so daß die Abplattung bereits meßbar ist und der Abstandswert D unterschritten wird, so kann die Steuereinheit 32 den Abstand d wieder vergrößern, bis die Situation d=D erreicht wird.

Der Punkt, an dem die Abplattung einsetzt, ist durch das Bildverarbeitungssystem 30 speicherbar und muß daher nicht bei jedem Meßzyklus aufs Neue bestimmt werden, sondern kann zum unmittelbaren Einstellen des Abstandswertes D aus dem Speicher abgerufen werden. so daß der Meßzyklus verkürzt wird.

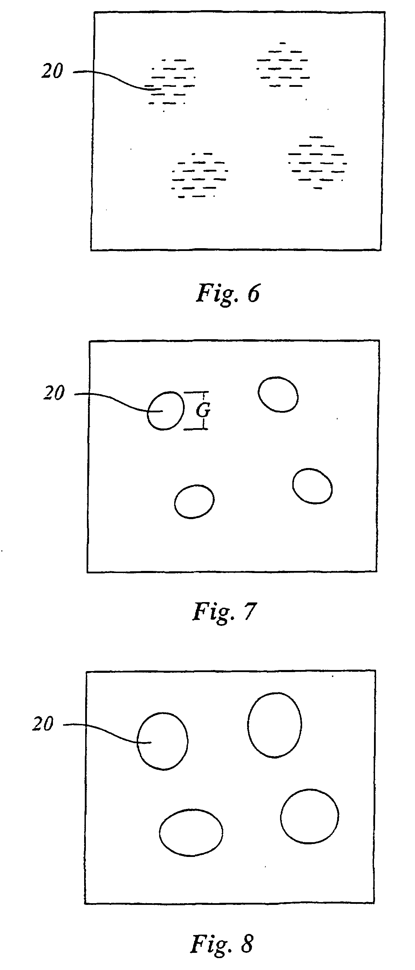

Die Fig. 6 bis 8 zeigen die Abbildungen der Zellen 20, die vom Bildaufnehmer 28 aufgenommen und von dem Bildverarbeitungssystem 30 verarbeitet werden, in der Weise, daß die einzelnen Fig. 5 bis 7 den Figuren 2 bis 4 zuzuordnen sind. Fig. 5 zeigt nur eine unscharfe Abbildung der Zellen 20, da diese sich außerhalb der Schärfenebene des Objektivs 18 befinden. Ist der Abstand D erreicht, an dem eine Lage von Zellen 20 genau zwischen den Fenstern 14, 16 liegt, befinden sich alle Zellen 20 in der Schärfenebene und werden deutlich abgebildet. In dieser Situation kann eine optische Analyse einzelner Zellparameter durchgeführt werden. Werden die Fenster 14,16 darüber hinaus weiter zusammengefahren, so werden die Zellen abgeplattet, wie es in Fig. 7 deutlich zu sehen ist. Der Durchmesser G steigt an, und diese Anwachsen kann durch ein entsprechend programmiertes Bildverarbeitungssystem 30 registriert werden, so daß die Dicke d des Probenvolumens 12 auf die oben beschriebene Weise eingestellt werden kann.

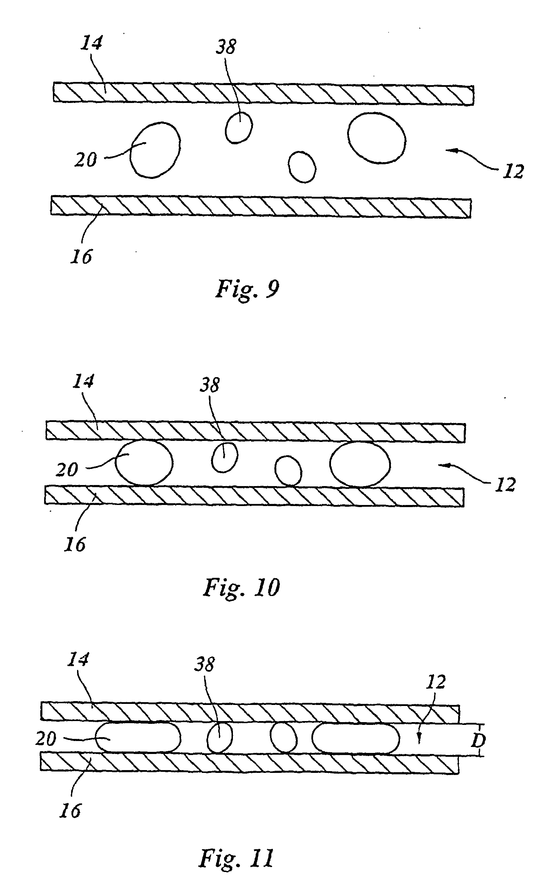

Die Figuren 9 bis 11 entsprechen den Figuren 2 bis 4, abgesehen davon, daß sich nun Zellen 20,38 unterschiedlicher Größen im Probenvolumen 12 befinden. Zur Einstellung des Abstandswerts D der Fenster 14.16 lassen sich in diesem Fall Zellen 38 vorgegebener Größe heranziehen, in der Weise, daß die Fenster 14,16 bis auf den Abstand D zusammengefahren werden, an welchem eine Abplattung der Zellen 38 der ausgewählten Größe stattfindet. Die übrigen Zellen 20 werden hierbei außer acht gelassen. Werden wie im vorliegenden Beispiel kleinere Zellen 38 zum Einstellen des Abstands D herangezogen, so werden die größeren Zellen 20 naturgemäß in sehr starkem Maße abgeplattet, bis der Zustand aus Fig. 11 erreicht wird. Eine starke Deformation oder eine Zerstörung der größeren Zellen 20 wird hierbei in Kauf genommen, da diese Zellen sich durch das Bildverarbeitungssystem leicht von den zu analysierenden Zellen 38 unterscheiden lassen. Beispielsweise werden die zerstörten Zellen 20 einfach vom untersuchten Abbildungsbereich subtrahiert. Möglich ist auch, die größeren Zellen 20 zur Einstellung des Abstands heranzuziehen, so daß die Fenster 14,16 nur bis zu dem Punkt zusammengefahren werden, an dem eine Abplattung der größten Zellen 20 einsetzt. Diese Situation entspricht etwa Fig. 10.

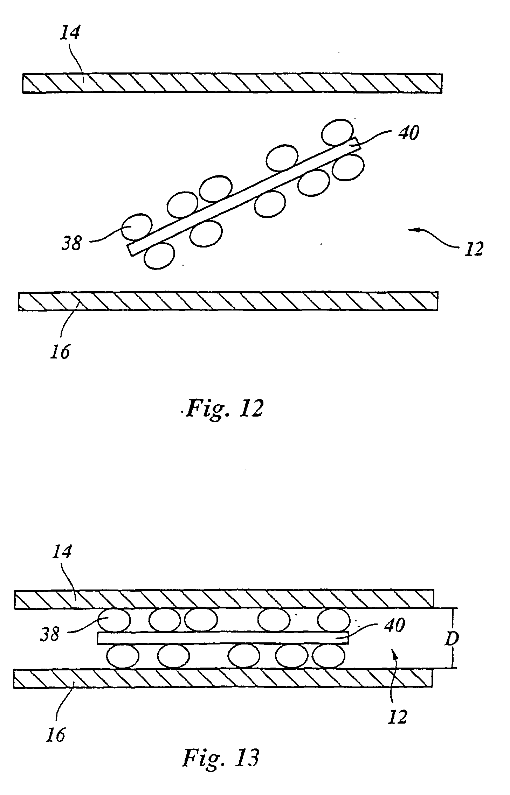

Fig. 12 zeigt ein Probenvolumen 12 mit einem frei schwimmenden Carrier 40, auf dem eine Anzahl von Zellen 38 anhaftet. Bei dem Carrier 40 handelt es sich um ein Polystyrolplättchen mit einem Durchmesser von 0,1 bis 0,2 mm und einer Dicke von 20 µm. Zur Beobachtung der anhaftenden Zellen 38 wird die Dikke d verringert, so daß sich der Carrier 40 gemäß Fig. 13 flach zwischen den Fenstern 14,16 anordnet und die Zellen 38 auf den gegenüberliegenden Oberflächenseiten durch den Anpreßdruck abgeflacht werden. Durch das beidseitige Anhaften von Zellen 38 auf der Oberfläche des Carriers 40 befinden sich jedoch in diesem Fall zwei Lagen von Zellen 38 im Probenvolumen 12. Zur Beobachtung der Zellen 38 ist es daher notwendig, die Schärfentiefe des Objektivs gering zu wählen und die Objektebene, d. h, den Bereich, der durch das Objektiv 18 scharf abgebildet wird, in eine dieser Zellagen zu verlagern. Es wird also in diesem Fall entweder die dem Objektiv zugewandte Zellage oder die auf der Rückseite des Carriers 40 befindliche Lage ausgewählt und abgebildet. Dies wird durch ein entsprechendes Verstellen des Mikroskopobjektivs 18 oder des Bildaufnehmers 28 erreicht. Die Schärfentiefe läßt sich durch Vergrößern der numerischen Apertur und durch Öffnen der Blende 36 verringern, so daß die jeweils vor oder hinter der abgebildeten Zellschicht liegende Zellage sich nicht störend auswirkt. Der Vorgang zum Einstellen der Dicke des Probenvolumens 12 entspricht im wesentlichen dem oben beschriebenen Fall, daß sich frei schwimmende Zellen in der Kulturflüssigkeit befinden, jedoch mit dem Unterschied, daß die Zellen 38 nicht unmittelbar zwischen den Fenstern 14,16, sondern zwischen einem der Fenster 14,16 und dem Carrier 40 liegen. An dem Punkt, an dem die Abplattung der Zellen 38 einsetzt, beträgt der Abstand D der Fenster 14,16 etwa der Dicke des Carriers 40 zuzüglich dem zweifachen Zelldurchmesser. Durch dieses Verfahren läßt sich beispielsweise die Bewuchsdichte und der Verwachsungsgrad der Zellen 38 auf den Carriern 40 leicht bestimmen.

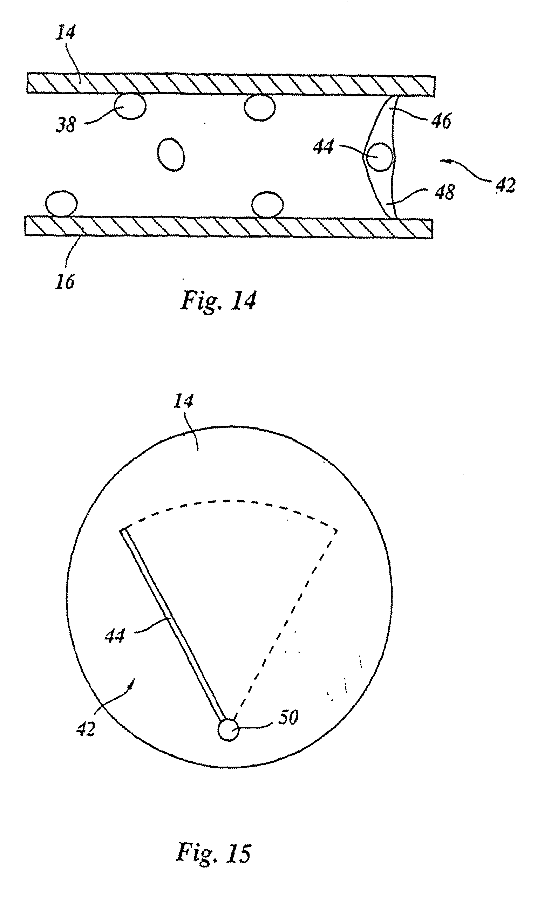

Fig. 14 zeigt ein Probenvolumen 12 mit einem Wischer 42, der Zellen 38, die nach dem Meßvorgang an den Fenstern 14,16 anhaften, von diesen entfernt. Dies ist deshalb vorteilhaft, weil die anhaftenden Zellen 38 bei jedem neuen Meßzyklus aufs Neue erfaßt werden und so die Meßergebnisse verfälscht werden. Es handelt sich bei dem Wischer 42 im wesentlichen um einen Arm 44 mit zwei gegenüberliegend an den Fenstern 14, 16 anliegenden Gummilippen 46,48 aus Silikongummi. Wie Fig. 15 zeigt, ist dieser Arm 44 an einem Ende an eine Schwenkachse 50 angebracht, die ihn in eine Hin- und Herbewegung zwischen den Fenstern 14,16 versetzt, so daß die Gummilippen 46,48 über die Oberflächen gezogen werden und die Fenster 14,16 reinigen. Die Bildverarbeitung 30 läßt sich so programmieren, daß der Erfolg des Wischvorgangs überprüft und dieser gegebenenfalls wiederholt wird.

In dem Fall, daß sich sowohl frei schwimmende Zellen als auch Carrier 40 in der Kulturflüssigkeit befinden, kann das Bildverarbeitungssystem 30 anhand der Abbildung eine Entscheidung darüber vornehmen, ob sich im Probenvolumen ein Carrier 40 oder ausschließlich frei schwimmende Zellen aufhalten, und es können vorgespeicherte Werte für den Abstand D entsprechend diesen beiden Fällen eingestellt werden.

## Patentansprüche

1. Verfahren zur Untersuchung von Zellen (20, 38) in einer Kulturflüssigkeit, insbesondere zur In-situ-Mikroskopie in einem Bioreaktor, durch mikroskopische Abbildung der Zellen innerhalb eines Probenvolumens (12), dessen Dicke (d) in Richtung der optischen Achse des Mikroskops (18) durch Fenster (14,16) begrenzt ist, und automatische Erfassung und Verarbeitung der Abbildung durch ein Bildverarbeitungssystem (30), wobei die Dikke (d) des Probenvolumens (12) der Größe der Zellen (20,38) angepaßt wird, indem der Abstand (d) der Fenster (14, 16) sukzessive verringert wird und gleichzeitig die Abbildungsgröße (G) der Zellen (20,38) durch das Bildverarbeitungssystem (30) kontrolliert wird, so daß ein Abstandswert (D) bestimmt wird, bei dem die Abbildungsgröße (G) der Zellen (20,38) entsprechend der durch den Berührungsdruck der Fenster (14,16) bewirkten Abplattung anzuwachsen beginnt, und daß der Abstand (d) der Fenster (14,16) zur Untersuchung auf diesen Abstandswert (D) eingestellt wird.

2. Verfahren gemäß Anspruch 1, **dadurch gekennzeichnet, daß** durch das Bildverarbeitungssystem (30) Zellen (20,38) verschiedener Größen klassifiziert werden und der Abstandswert (D) bestimmt wird, bei dem die Abbildungsgröße (G) der Zellen (20,38) einer vorbestimmten Größenklasse anzuwachsen beginnt, und daß der Abstand (d) zur Untersuchung auf diesen Abstandswert (D) eingestellt wird.

3. Verfahren gemäß Anspruch 1, **dadurch gekennzeichnet, daß** zur Untersuchung von an plättchenförmigen Carriern (40) anhaftenden Zellen (38) der Abstandswert (D) bestimmt wird, bei dem die Abbildungsgröße (G) der Zellen (38) auf einer Oberflächenseite eines flach zwischen den Fenstern (14,16) liegenden Carriers (40) anzuwachsen beginnt, und daß der Abstand (d) zur Untersuchung auf diesen Abstandswert (D) eingestellt wird.

4. Verfahren gemäß Anspruch 3, **dadurch gekennzeichnet, daß** zur Untersuchung von Zellen (38) auf einer vorbestimmten Oberflächenseite des Carriers (40) die abzubildende Objektebene des Mikroskopobjektivs (18) in die zu untersuchende Zellschicht verlagert wird und die Schärfentiefe so gering gewählt wird. daß ausschließlich Zellen (38) unmittelbar in der Objektebene von der Bildverarbeitung (30) erfaßt werden.

5. Verfahren gemäß Anspruch 4, **dadurch gekennzeichnet, daß** die Schärfentiefe durch Verändern der numerischen Apertur des Mikroskopobjektivs (18) verändert wird.

6. Verfahren gemäß einem der vorhergehenden Ansprüche, **dadurch gekennzeichnet, daß** der in einem Meßzyklus bestimmte Abstandswert (D) gespeichert wird und bei nachfolgenden Meßzyklen der Abstand (d) der Fenster (14,16) unmittelbar auf den gespeicherten Wert (D) eingestellt wird.

7. Verfahren gemäß Anspruch 6 in Verbindung mit Anspruch 3, 4 oder 5, **dadurch gekennzeichnet, daß** durch das Bildverarbeitungssystem (30) eine Entscheidung darüber getroffen wird, ob sich ein Carrier (40) oder eine Zelle (20,38) im Probenvolumen (12) befindet, und daß der Abstand (d) der Fenster (14,16) auf Grundlage dieser Entscheidung auf einen gespeicherten Abstandswert (D) für einen Carrier (40) oder eine Zelle (20,38) eingestellt wird.

8. Verfahren gemäß einem der vorhergehenden Ansprüche, **dadurch gekennzeichnet, daß** zumindest eines der Fenster (14,16) vor oder nach dem Meßvorgang durch einen Wischer (42) gereinigt wird.

9. Vorrichtung zur Untersuchung von Zellen (20,38) in einer Kulturflüssigkeit, insbesondere zur In-situ-Mikroskopie in einem Bioreaktor, mit einem Mikroskop (18) zur Abbildung von Zellen (20,38) innerhalb eines Probenvolumens (12), das in Richtung der optischen Achse des Mikroskops (18) durch Fenster (14,16) begrenzt ist, und ein Bildverarbeitungssystem (30) zur Erfassung und Verarbeitung der Mikroskop-Abbildung, wobei eine Verstelleinheit (34) zum Verstellen des Abstands (d) der Fenster (14,16) des Probenvolumens (12) entlang der optischen Achse, die mittels einer Steuereinheit (32) durch das Bildverarbeitungssystem (30) auf solche Weise ansteuerbar ist. daß der Abstand (d) der Fenster (14,16) zur Untersuchung auf einen Abstandswert (D) einstellbar ist, bei dem die Abbildungsgröße (G) der Zellen (20,38) entsprechend der durch den Berührungsdruck der Wände (14, 16) bewirkten Abplattung anzuwachsen beginnt.

10. Vorrichtung gemäß Anspruch 9, **dadurch gekennzeichnet, daß** das Bildverarbeitungssystem (30) zur Speicherung des bestimmten Abstandswertes (D) vorgesehen ist, der zum Einstellen des Abstandes (d) auf einen vorbestimmten Abstandswert (D) abrufbar ist.

11. Vorrichtung gemäß Anspruch 10 oder 11, **gekennzeichnet durch** eine Blende (36) zur Einstellung der Schärfentiefe des Mikroskops (18).

12. Vorrichtung gemäß Anspruch 10, 11 oder 12, **gekennzeichnet durch** einen Wischer (42) zum Reinigen der Fenster (14,16) des Probenvolumens (12).

## Claims

1. A method for the examination of cells (20, 38) in a culture medium, in particular for in-situ microscopy in a bio-reactor, through the microscopic imaging of cells in a sample volume (12), the depth (d) of which is defined by windows (14, 16) in the direction of the optical axis of the microscope (18), and the automatic recording and processing of the image by means of an image processing system (30), whereby the depth (d) of the sample volume (12) is adjusted to the size of the cells (20, 38) by successively reducing the separation (d) of the windows while the image size (G) of the cells (20, 38) is simultaneously verified by the image processing system (30) such that a separation value (D) is determined at which the image size (G) of the cells begins to grow, thus corresponding to flattening caused by the contact pressure of the windows (14, 16), and that the separation (d) of the windows (14, 16) is set to said separation value (D) for the examination.

2. Method according to Claim 1, **characterized in that** the image processing system (30) classifies cells (20, 38) into different size categories and determines the separation value (D) as that point where the image size (G) of the cells (20, 38) of a pre-determined size category begins to grow, and that the separation (d) is set to this separation value (D) for the examination.

3. Method according to Claim 1, **characterized in that**, for the examination of cells (38) adhering to platelet-shaped carriers (40), the separation value (D) is determined where the image size (G) of the cells (38) on a surface side of a carrier lying flat between the windows (14, 16) begins to grow, and that the separation (d) is set to this separation value (D) for the examination.

4. Method according to Claim 3, **characterized in that**, for the examination of cells (38) on a predetermined surface side of the carrier (40), the object level of the microscope lens (81) to be imaged is shifted to the cell layer to be examined, with the depth of field being selected so low that exclusively cells (38) lying immediately in the object layer are recorded by the image processing system (30).

5. Method according to Claim 4, **characterized in that the** depth of field is altered by adjusting the numeric aperture of the microscope lens (18).

6. Method according to one of the preceding Claims, **characterized in that** the separation value (D) determined in one measuring cycle is stored and in the subsequent measuring cycles the separation (d) of the windows (14, 16) is set immediately to the stored value (D).

7. Method according to Claim 6 in conjunction with Claim 3, 4 or 5, **characterized in that** an assessment is made by the image processing system (30) as to whether a carrier (40) or a cell (20, 38) is present in the sample volume (12), and that on the basis of this assessment the separation (d) of the windows (14, 16) is set to a stored separation value (D) for a carrier (40) or for a cell (20, 38).

8. Method according to one of the preceding Claims, **characterized in that** at least one of the windows (14, 16) is cleaned by a wiper (42) before or after a measurement is made.

9. An apparatus for the examination of cells (20, 38) in a culture medium, in particular for in-situ microscopy in a bio-reactor, with a microscope (18) for the imaging of cells (20, 38) within a sample volume (12), which is defined by windows (14, 16) in the direction of the optical axis of the microscope (18), and an image processing system (30) for recording and processing the microscope image, whereby an actuator (34) for adjusting the separation (d) between the windows (14, 16) of the sample volume (12) along the optical axis which can be controlled by the image processing system (30) by means of a control unit (32) such that the separation (d) between the windows (14, 16) can be set for the examination to a separation value (D) at which point the image size (G) of the cells (14, 16) starts to grow in accordance with their flattening caused by the contact pressure of the windows (14, 16).

10. Apparatus according to Claim 9, **characterized in that** the image processing system (30) is provided for the storage of the determined separation value (D), which can be retrieved for setting the separation (d) to a pre-determined separation value (D).

11. Apparatus according to Claim 10 or 11, **characterized by** a diaphragm (36) for setting the depth of field of the microscope.

12. Apparatus according to Claim 10, 11 or 12, **characterized by** a wiper (42) for cleaning the windows (14, 16) of the sample volume (12).

## Revendications

1. Procédé d'examen de cellules (20, 38) dans un liquide de culture, en particulier de microscopie in situ dans un bioréacteur, par formation au microscope d'images des cellules à l'intérieur d'un volume échantillon (12) dont l'épaisseur (d) dans la direction de l'axe optique du microscope (18) est limitée par des fenêtres (14, 16), et saisie et traitement automatiques de l'image par un système de traitement d'images (30), l'épaisseur (d) du volume échantillon (12) étant adaptée à la grandeur des cellules (20, 38) par diminutions successives de la distance (d) des fenêtres (14, 16) et en même temps contrôle de la grandeur (G) de l'image des cellules (20, 38) par le système de traitement d'images (30), de sorte qu'est déterminée une valeur de distance (D) pour laquelle la grandeur (G) de l'image des cellules (20, 38) commence à croître en fonction de l'aplatissement produit par la pression de contact des fenêtres (14, 16), et que la distance (d) des fenêtres (15, 16) est fixée pour l'examen à cette valeur de distance (D).

2. Procédé selon la revendication 1, **caractérisé par le fait que** le système de traitement d'images (30) classe des cellules (20, 38) de différentes grandeurs et détermine la valeur de distance (D) pour laquelle la grandeur (G) de l'image des cellules (20, 38) d'une catégorie déterminée de grandeurs commence à croître, et que la distance (d) est fixée pour l'examen à cette valeur de distance (D).

3. Procédé selon la revendication 1, **caractérisé par le fait que** pour l'examen de cellules (38) qui adhèrent à des supports en forme de plaquette (40) est déterminée la valeur de distance (D) pour laquelle la grandeur (G) de l'image des cellules (38) commence à croître sur une face d'un support (40) situé à plat entre les fenêtres (14, 16), et que la distance (d) est fixée pour l'examen à cette valeur de distance (D).

4. Procédé selon la revendication 3, **caractérisé par le fait que** pour l'examen de cellules (38) sur une face déterminée du support (40), le plan objet dont il s'agit de former l'image de l'objectif (18) du microscope est amené dans la couche de cellules à examiner et la profondeur de foyer est choisie suffisamment petite pour que le système de traitement d'images (30) saisisse seulement des cellules (38) directement dans le plan objet.

5. Procédé selon la revendication 4, **caractérisé par le fait que** la profondeur de foyer est modifiée par modification de l'ouverture numérique de l'objectif (18) du microscope.

6. Procédé selon l'une des revendications précédentes, **caractérisé par le fait que** la valeur de distance (D) déterminée dans un cycle de mesures est mise en mémoire, et, pour les cycles de mesures suivants, la distance (d) des fenêtres (14, 16) est fixée directement à la valeur (D) mise en mémoire.

7. Procédé selon la revendication 6 en association avec l'une des revendications 3, 4 et 5, **caractérisé par le fait que** le système de traitement d'images (30) décide si un support (40) ou une cellule (20, 38) se trouve dans le volume échantillon (12), et que la distance (d) des fenêtres (14, 16) est fixée d'après cette décision à une valeur de distance (D) en mémoire pour un support (40) ou une cellule (20, 38).

8. Procédé selon l'une des revendications précédentes, **caractérisé par le fait qu'**au moins une des fenêtres (14, 16) est, avant ou après l'opération de mesure, nettoyée par un dispositif d'essuyage (42).

9. Dispositif pour l'examen de cellules (20, 38) dans un liquide de culture, en particulier la microscopie in situ dans un bioréacteur, comportant un microscope (18) pour la formation d'images de cellules (20, 38) à l'intérieur d'un volume échantillon (12) qui est limité dans la direction de l'axe optique du microscope (18) par des fenêtres (14, 16), et un système de traitement d'images (30) pour la saisie et le traitement de l'image formée par le microscope, dans lequel est prévu un dispositif de réglage (34) pour le réglage de la distance (d) des fenêtres (14, 16) du volume échantillon (12) le long de l'axe optique qui peut être commandé au moyen d'un dispositif de commande (32) par le système de traitement d'images (30) de façon telle que la distance (d) des fenêtres (14, 16) puisse être fixée pour l'examen à une valeur de distance (D) pour laquelle la grandeur (G) de l'image des cellules (20, 38) commence à croître en fonction de l'aplatissement produit par la pression de contact des parois (14, 16).

10. Dispositif selon la revendication 9, **caractérisé par le fait que** le système de traitement d'images (40) est prévu pour la mise en mémoire de la valeur de distance déterminée (D), qui peut être appelée pour le réglage de la distance (d) à une valeur prédéterminée (D).

11. Dispositif selon l'une des revendications 9 et 10, **caractérisé par** un diaphragme (36) pour le réglage de la profondeur de foyer du microscope (18).

12. Dispositif selon l'une des revendications 9, 10 et 11, **caractérisé par** un dispositif d'essuyage (42) pour le nettoyage des fenêtres (14, 16) du volume échantillon (12).
